(19) Europäisches Patentamt
European Patent Office
Office européen des brevets

(11) EP 3 900 612 A1

(12) **EUROPÄISCHE PATENTANMELDUNG**

(43) Veröffentlichungstag:
27.10.2021 Patentblatt 2021/43

(21) Anmeldenummer: 20171431.8

(22) Anmeldetag: **24.04.2020**

(51) Int Cl.:
*A61B 5/00* (2006.01)   *A61B 5/0205* (2006.01)
*G16H 10/00* (2018.01)   *A61F 13/00* (2006.01)
*H01M 4/00* (2006.01)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **VARTA Microbattery GmbH
73479 Ellwangen (DE)**

(72) Erfinder:
• **Rieger, Joachim
73433 Aalen (DE)**
• **Krebs, Martin
73494 Rosenberg (DE)**

(74) Vertreter: **Patentanwaltskanzlei Cartagena
Partnerschaftsgesellschaft Klement, Eberle mbB
Urbanstraße 53
70182 Stuttgart (DE)**

(54) **DIAGNOSTISCHES PFLASTER**

(57) Ein diagnostisches Pflaster (10) umfasst ein flächiges Substrat (11) zur Aufbringung auf die Körperoberfläche eines Probanden. Weiterhin umfasst das Pflaster (10) mindestens eine Sensorik-Einrichtung (14), mindestens eine Einrichtung (15) zur drahtlosen Datenübertragung und mindestens ein gedrucktes Energiespeicherelement (16).

Fig. 1A

Fig. 1B

**Beschreibung**

Die vorliegende Erfindung betrifft ein diagnostisches Pflaster sowie ein Verfahren zur Herstellung eines solchen Pflasters.

In der medizinischen Diagnostik ist es seit langem bekannt, verschiedene Körperfunktionen eines Probanden mit Hilfe von Sensoren, die direkt am Körper angebracht werden, zu überwachen. Auf diese Weise können beispielsweise der Puls, die Sauerstoffsättigung im Blut oder die Temperatur im Körper des Probanden beobachtet werden.

Es ist auch bereits bekannt, eine geeignete Sensorik mittels eines auf die Haut aufklebbaren Pflasters an dem Probanden anzubringen. So beschreibt beispielsweise die DE 20 2016 003 095 U1 ein Puls-Oxymeter-Messgerät in Pflasterform, das die für eine Pulsoxymetrie nötigen physikalischen Komponenten enthält sowie zusätzlich die für die Ansteuerung und Signalgewinnung nötige periphere Elektronik aufweist. Zusätzlich kann in dem Pflaster ein Energiespeicherelement enthalten sein, bei dem es sich um eine Folienbatterie, eine Lithium-Zelle oder einen Lithium-Akkumulator handeln kann.

Die EP 1 062 906 A1 beschreibt eine Vorrichtung zur medizinischen Langzeitüberwachung von beispielsweise Astronauten. Die Vorrichtung besteht aus mindestens einer autonomen Sensoreinheit, einer zentralen Sende- und Empfangseinheit sowie einer portablen Datenaufzeichnungseinheit. Dabei ist die Sensoreinheit auf einem Sensorträger angeordnet, der als Pflaster ausgebildet ist. Ferner enthält die Sensoreinheit einen Mikrochip, der mit einer Batterie als Energiespeicher versehen sein kann.

Allgemein ist die Energieversorgung von derartigen Sensorpflastern nicht unproblematisch. Zum einen sollen die eingesetzten Energiespeicherelemente leicht und klein sein, um bei der Anbringung mittels eines Pflasters am Körper des Probanden nicht zu stören. Zum anderen sollten sie kostengünstig herstellbar sein und insbesondere auch für einen Einwegartikel geeignet sein. Schließlich sollte von den verwendeten Energiespeicherelementen keine gesundheitliche Gefahr für den Probanden ausgehen. So sind beispielsweise Lithium-Zellen insbesondere im Hinblick auf die darin enthaltenen Elektrolytlösungen gesundheitlich nicht unbedenklich. Zudem geht von derartigen Energiespeicherelementen eine nicht unerhebliche Entzündungs- und Brandgefahr aus.

Um derartige Nachteile und Probleme von aus dem Stand der Technik bekannten Sensorpflastern zu vermeiden, stellt sich die Erfindung die Aufgabe, ein diagnostisches Pflaster bereitzustellen, das insbesondere im Hinblick auf das eingesetzte EnergiespeicherelementVerbesserungen im Vergleich mit herkömmlichen Sensorpflastern bietet. Insbesondere soll das diagnostische Pflaster mit einer optimalen Energieversorgung ausgestattet sein, von der weder Unbequemlichkeiten noch Gefahren ausgehen und die zudem insbesondere im Hinblick auf den Einsatz eines diagnostischen Pflasters als Einwegartikel besonders kostengünstig herstellbar ist.

Diese Aufgabe wird durch ein diagnostisches Pflaster mit den Merkmalen des Anspruchs 1 gelöst. Weiterhin wird diese Aufgabe durch ein Verfahren zur Herstellung eines diagnostischen Pflasters gemäß dem weiteren unabhängigen Anspruch gelöst. Bevorzugte Ausgestaltungen dieses diagnostischen Pflasters bzw. des Verfahrens zur Herstellung eines solchen Pflasters ergeben sich aus den abhängigen Ansprüchen.

Das erfindungsgemäße diagnostische Pflaster zeichnet sich durch die folgenden Merkmale aus:

    a. das Pflaster umfasst ein flächiges Substrat zur Aufbringung auf die Körperoberfläche eines Probanden,
    b. das Pflaster umfasst mindestens eine Sensorik-Einrichtung,
    c. das Pflaster umfasst mindestens eine Einrichtung zur drahtlosen Datenübertragung,
    d. das Pflaster umfasst mindestens ein gedrucktes Energiespeicherelement.

Bevorzugt sind die Sensorik-Einrichtung, die Einrichtung zur drahtlosen Datenübertragung und das gedruckte Energiespeicherelement nebeneinander oder überlappend auf dem flächigen Substrat angeordnet.

Im Unterschied zu herkömmlichen, vergleichbaren Sensorpflastern weistdas erfindungsgemäße diagnostische Pflaster mindestens ein gedrucktes Energiespeicherelement auf. Der Einsatz eines gedruckten Energiespeicherelements hat dabei den besonderen Vorteil, dass ein derartiges Energiespeicherelement in miniaturisierter und besonders platzsparender Weise auf dem Pflaster angebrachtwerden kann, so dass das Pflaster genügend Raum für die übrigen erforderlichen Komponenten des diagnostischen Pflasters bereithalten kann.

Darüber hinaus erlaubt ein gedrucktes Energiespeicherelement eine vergleichsweise hohe Flexibilität und Biegbarkeit des Energiespeicherelements, was im Zusammenhang mit dem diagnostischen Pflaster bei der Anbringung des Pflasters auf der Körperoberfläche des Probanden besondere Vorteile, beispielsweise im Hinblick auf einen Tragekomfort, bietet. Weiterhin können gedruckte Energiespeicherelemente in besonders kostengünstiger Weise hergestellt werden, so dass das erfindungsgemäße diagnostische Pflaster auch in wirtschaftlicher Hinsicht besonders vorteilhaft ist. Das erfindungsgemäße diagnostische Pflaster kann prinzipiell an beliebiger Stelle am Körper des Probanden angebracht werden. Besonders bevorzugt ist eine Anbringung am Körperstamm, beispielsweise im Brustbereich oder Bauchbereich des Probanden.

Beschreibung des Energiespeicherelements

Unter einem Energiespeicherelement werden vorliegend sowohl eine einzelne zur Speicherung elektrischer Energie befähigte elektrochemische Zelle als auch eine Batterie mit mehreren elektrisch miteinander verschalteten, zur Speicherung elektrischer Energie befähigten elektrochemischen Zellen verstanden. Jede elektrochemische Zelle und damit jedes Energiespeicherelement im Sinne der vorliegenden Anmeldung umfasst mindestens eine positive und mindestens eine negative Elektrode.

In elektrochemischen Zellen findet eine elektrochemische, energieliefernde Reaktion statt, welche sich aus zwei elektrisch miteinander gekoppelten, aber räumlich voneinander getrennten Teilreaktionen zusammensetzt. Eine bei vergleichsweise niedrigerem Redoxpotential stattfindende Teilreaktion läuft an der negativen Elektrode ab. Eine weitere Teilreaktion läuft bei vergleichsweise höherem Redoxpotential an der positiven Elektrode ab. Bei der Entladung werden an der negativen Elektrode durch einen Oxidationsprozess Elektronen freigesetzt, resultierend in einem Elektronenstrom über einen äußeren Verbraucher zur positiven Elektrode, von der eine entsprechende Menge an Elektronen aufgenommen wird. An der positiven Elektrode findet also ein Reduktionsprozess statt. Zeitgleich kommt es zum Zwecke des Ladungsausgleichs zu einem der Elektrodenreaktion entsprechenden Ionenstrom innerhalb des Energiespeicherelements. Dieser Ionenstrom wird durch einen ionenleitenden Elektrolyten gewährleistet.

Wenn der Entladeprozess reversibel ist, spricht man bei solchen wiederaufladbaren elektrochemischen Zellen auch von sekundären Zellen im Gegensatz zu primären Zellen, die nicht wiederaufladbar sind. Wenn im Zusammenhang mit sekundären elektrochemischen Zellen die Begriffe "Anode" und "Kathode" benutzt werden, benennt man die Elektroden in der Regel entsprechend ihrer Entladefunktion. Die negative Elektrode in solchen Energiespeicherelementen ist also die Anode, die positive Elektrode die Kathode.

Bei dem erfindungsgemäßen diagnostischen Pflaster ist es besonders bevorzugt, primäre, also nicht wiederaufladbare Energiespeicherelemente zu verwenden, da das diagnostische Pflaster vorzugsweise als Einwegartikel eingesetzt wird und nur für einen einmaligen Gebrauch bestimmt ist. Hierbei erübrigt sich also eine Wiederaufladung des Energiespeicherelements. Prinzipiell kann das erfindungsgemäße diagnostische Pflaster jedoch auch ein sekundäres Energiespeicherelement aufweisen.

Die negative Elektrode und die positive Elektrode des Energiespeicherelements umfassen jeweils eine elektrochemisch aktive Komponente (Elektrodenaktivmaterial), welche jeweils unmittelbar mit einem elektrisch leitenden Stromleiter in Kontakt steht, der in der Regel flächig ausgestaltet ist. Die elektrisch leitenden Stromleiter gewährleisten die elektrische Anbindung des Energiespeicherelements an den jeweiligen Verbraucher, insbesondere an die Sensorik-Einrichtung und/oder an die Einrichtung zur drahtlosen Datenübertragung des diagnostischen Pflasters. Beispielsweise kann das Elektrodenaktivmaterial auf den flächigen Stromleiter aufgedruckt sein.

Darüber hinaus können die Elektroden jeweils elektrochemisch inaktive Komponenten enthalten. Hierzu zählen insbesondere Leitfähigkeitadditive und ein elastisches Bindemittel oder Bindemittelgemisch als Elektrodenbinder.

Unter einem gedruckten Energiespeicherelement wird vorliegend insbesondere ein Energiespeicherelement verstanden, bei dem zumindest die Elektroden, in einigen bevorzugten Ausführungsformen auch die elektrischen Stromleiter und/oder ein gegebenenfalls vorgesehener Separator, durch Drucken einer Druckpaste auf einen Träger, insbesondere mittels eines Siebdruckverfahrens, gebildet sind.

In besonders bevorzugten Ausgestaltungen des erfindungsgemäßen diagnostischen Pflasters weist das diagnostische Pflaster im Hinblick auf das gedruckte Energiespeicherelement mindestens eines der unmittelbar folgenden zusätzlichen Merkmale a. bis c. auf:

    a. das gedruckte Energiespeicherelement umfasst eine negative Elektrode, die als Elektrodenaktivmaterial metallisches Zink oder eine metallische Zinklegierung aufweist,

    b. das gedruckte Energiespeicherelement umfasst eine positive Elektrode, die als Elektrodenaktivmaterial mindestens ein Metalloxid und/oder einen Katalysator, der eine Reduktion von Luftsauerstoff ermöglicht, aufweist,

    c. das gedruckte Energiespeicherelement umfasst einen wässrigen Elektrolyten.

In besonders bevorzugter Weise sind bei dem diagnostischen Pflaster sowohl die unmittelbar vorstehenden Merkmale a. und b. als auch c. in Kombination miteinander verwirklicht.

In diesen bevorzugten Ausgestaltungen handelt es sich bei dem Energiespeicherelement um eine sogenannte Zink-Metalloxid-Zelle oder um eine Zink-Luft-Zelle. Die Wahl eines elektrochemischen Systems mit einer zinkhaltigen negativen Elektrode ist vor allem im Hinblick auf die Sicherheit sehr vorteilhaft. Systeme mit zinkbasierten negativen Elektroden können mit einem wässrigen Elektrolyten arbeiten und sind damit nicht brennbar. Darüber hinaus ist Zink umweltverträglich und billig.

Diese Ausgestaltung des erfindungsgemäßen diagnostischen Pflasters hat im Vergleich mit herkömmlichen, vergleichbaren Sensorpflastern damit den besonderen Vorteil, dass die Verwendung von Lithium-Zellen vermieden wird. Aufgrund der in Lithium-Zellen verwendeten Substanzen geht von derartigen Energiespeicherelementen eine erhebliche Gesundheitsgefahr aus. Dies wird bei dem erfindungsgemäßen diagnostischen Pflaster vermieden, indem die beschriebenen Zink-Metalloxid-Zellen oderZink-Luft-Zellen als Energiespeicherelemente eingesetzt werden. Die bei diesen Zellen verwendeten Substanzen sind in der Regel gesundheitlich wenig bedenklich. Zudem geht von diesen bevorzugten Energiespeicherelementen auch aufgrund des wässrigen Elektrolyten keinerlei Entzündungs- und/oder Brandgefahr aus, so dass mit diesen elektrochemischen Zellen ausgestattete diagnostische Pflaster besonders vorteilhaft sind.

Bei dem Energiespeicherelement des erfindungsgemäßen diagnostischen Pflasters sind in dieser Ausführungsform vorzugsweise zumindest die zinkhaltige negative Elektrode, die metalloxidhaltige positive Elektrode und die elektrischen Leiter zur Kontaktierung der Elektroden gedruckt.

Die negative Elektrode und gegebenenfalls die positive Elektrode können beispielsweise jeweils bevorzugt eine Dicke im Bereich von 10 $\mu$m bis 250 $\mu$m aufweisen.

Zink-Metalloxid-Zelle

In einer besonders bevorzugten Ausgestaltung des erfindungsgemäßen diagnostischen Pflasters handelt es sich bei dem Energiespeicherelement um eine Zink-Metalloxid-Zelle, die durch mindestens eines der unmittelbar folgenden zusätzlichen Merkmale a. bis f. gekennzeichnet ist:

    a. die negative Elektrode umfasst als Elektrodenbinder ein erstes elastisches Bindemittel oder Bindemittelgemisch,

    b. die positive Elektrode umfasst als Elektrodenbinder ein zweites elastisches Bindemittel oder Bindemittelgemisch,

    c. das Metalloxid der positiven Elektrode ist Manganoxid und/oder Silberoxid,

    d. die positive Elektrode umfasst mindestens ein Leitfähigkeitsadditiv, insbesondere ein Kohlenstoffbasiertes Leitfähigkeitsadditiv,

    e. der wässrige Elektrolyt ist ein wässriger Elektrolyt, insbesondere mit einem pH-Wert im Bereich von 6-8,

    f. der wässrige Elektrolyt umfasst mindestens ein chloridbasiertes Leitsalz, insbesondere Zinkchlorid und/oder Ammoniumchlorid.

Hierbei ist eine Kombination aller unmittelbar vorgenannten Merkmale a. bis f. besonders bevorzugt.

In einer ersten, besonders bevorzugten Variante kann das Energiespeicherelement, insbesondere ein derartiges Energiespeicherelement, in Form eines Schichtstapels ausgestaltet sein. Vorzugsweise weist ein solches Energiespeicherelement die unmittelbar folgenden Merkmale a. bis c. auf:

    a. Es umfasst einen Schichtstapel mit

    •  einer als Schicht ausgebildeten Anode, die in vorzugsweise homogener Durchmischung als Elektrodenaktivmaterial Partikel des metallischen Zinks oder der metallischen Zinklegierung aufweist und das erste elastische Bindemittel oder Bindemittelgemisch enthält, und

    •  einer als Schichtausgebildeten Kathode, die bevorzugt in homogener Durchmischung als Elektrodenaktivmaterial Partikel des mindestens einen partikulären Metalloxids, das mindestens ein Leitfähigkeitsadditiv zur Optimierung der elektrischen Leitfähigkeit der Kathode, und das zweite elastische Bindemittel oder Bindemittelgemisch enthält, und

    •  einem als Schicht ausgebildeten Separator, der die Anode und die Kathode elektrisch voneinander isoliert und zwischen der Anode und der Kathode angeordnet ist, und

    b. es umfasst einen ersten elektrischen Leiter, der mit der Anode in unmittelbarem Kontakt steht, sowie einen zweiten elektrischen Leiter, der mit der Kathode in unmittelbarem Kontakt steht, und

    c. es umfasst ein Gehäuse, das den Schichtstapel umschließt,

Hierbei ist eine Kombination aller unmittelbar vorgenannten Merkmale a. bis c. besonders bevorzugt.

In einer besonders bevorzugten Weiterbildung zeichnet sich ein derartiges Energiespeicherelement weiterhin durch die unmittelbar folgenden Merkmale a. und b. aus:

    a. der Separator umfasst eine erste und eine zweite Seite, von denen die erste Seite eine erste Kontaktfläche zur Anode und die zweite Seite parallel dazu eine zweite Kontaktfläche zur Kathode umfasst, und

    b. die Kontaktflächen überlagern einander in Blickrichtung senkrecht zu dem als Schicht ausgebildeten Separator in einem Überlagerungsbereich A, in welcher eine Gerade senkrecht zu dem Separator beide Kontaktflächen schneidet.

Prinzipiell eignet sich für das Energiespeicherelement des erfindungsgemäßen diagnostischen Pflasters auch eine koplanare Anordnung der Elektroden, die weiter unten noch näher erläutert wird. Die gestapelte Anordnung der Elektroden und des Separators kann jedoch besonders vorteilhaft sein, da die Strombelastbarkeit von Zellen mit gestapelten Elektroden im allgemeinen deutlich höher ist, da die Ionen, die bei Lade- und Entladevorgängen zwischen den Elektroden hin- und herwandern, im Mittel deutlich kürzere Wege zurücklegen müssen. Im Bereich des Überlagerungsbereichs entspricht die kürzeste Entfernung zwischen den Elektroden der Dicke des zwischen der negativen Elektrode (Anode) und der positiven Elektrode (Kathode) angeordneten Separators.

Bei identischer Größe und nicht versetzter Anordnung der negativen Elektrode und der positiven Elektrode innerhalb des Stapels entspricht die Größe des Überlagerungsbereichs exakt der Größe der Elektroden.

In besonders bevorzugten Ausgestaltungen kann sich das Energiespeicherelement weiterhin durch mindestens eines der unmittelbar folgenden Merkmale a. bis d., die mit einer besonderen Strombelastbarkeit einhergehen, auszeichnen:

a. Der Überlagerungsbereich A weist eine Mindestgröße von 0,5 cm$^2$, bevorzugt von 1 cm$^2$, weiter bevorzugt von 2 cm$^2$, besonders bevorzugt von 4 cm$^2$, auf.
b. Die Kathode enthält das partikuläre Metalloxid, bezogen auf das Gesamtgewicht der festen Bestandteile der Kathode, in einem Anteil von 10 Gew.-% bis 90 Gew.-%, und
c. Die Kathode enthält das zweite elastische Bindemittel oder Bindemittelgemisch, bezogen auf das Gesamtgewicht der festen Bestandteile der Kathode, in einem Anteil von 1 Gew.-% bis 25 Gew.-%, und
d. Die Kathode enthält das mindestens eine Leitfähigkeitsadditiv, bezogen auf das Gesamtgewicht der festen Bestandteile der Kathode, in einem Anteil von 1 Gew.-% bis 85 Gew.-%.

In bevorzugten Ausführungsformen können alle unmittelbar vorstehenden Merkmale a. bis d. in Kombination miteinander verwirklicht sein.

Der Überlagerungsbereich A weist bevorzugt eine Maximalgröße von 50 cm$^2$, besonders bevorzugt von 10 cm$^2$, auf.

In einer zweiten, besonders bevorzugten Variante kann das Energiespeicherelement die Anode und die Kathode in koplanarer Anordnung umfassen. Die Vorteile eines solchen Energiespeicherelements, bei dem die negative Elektrode und die positive Elektrode in einer Ebene nebeneinanderliegen, ist die geringe Dicke, die aus der koplanaren Anordnung resultiert. Allerdings können hierbei im Allgemeinen nur relativ schwache Stromstärken realisiert werden.

In besonders bevorzugter Weise wird daher das gedruckte Energiespeicherelement gemäß der obigen ersten Variante gebildet. Mit einem solchen Energiespeicherelement kann in besonders bevorzugter Weise der insbesondere für eine drahtlose Datenübertragung erforderliche Energiebedarf des entsprechend ausgestatteten diagnostischen Pflasters gedeckt werden.

Grundsätzlich beziehen sich alle prozentualen Angaben zu Gewichtsanteilen von Komponenten in Elektroden in der vorliegenden Anmeldung auf das Gesamtgewicht der festen Bestandteile der jeweiligen Elektrode. Die Gewichtsanteile der jeweils beteiligten Komponenten ergänzen sich dabei auf 100 Gew.-%. Vor ihrer Ermittlung ist gegebenenfalls in den Elektroden enthaltene Feuchtigkeit zu entfernen.

Der Anteil an dem zweiten elastischen Bindemittel oder Bindemittelgemisch beträgt vorzugsweise mindestens 1 Gew.-%, da es die in der Kathode enthaltenen Metalloxid partikel relativ zueinander fixieren und der Kathode gleichzeitig eine gewisse Flexibilität verleihen soll. Der Anteil überschreitet vorteilhafterweise den oben genannten Maximalanteil aber nicht, da ansonsten die Gefahr besteht, dass die Metalloxidpartikel zumindest teilweise nicht mehr in Kontakt zueinander stehen. Innerhalb des oben genannten Bereiches ist ein Anteil im Bereich von 1 Gew.-% bis 15 Gew.-%, besonders bevorzugt in einem Anteil von 5 Gew.-% bis 15 Gew.-%, weiter bevorzugt.

Die Anteile an dem partikulären Metalloxid und dem mindestens einen Leitfähigkeitsadditiv bedingen sich gegenseitig. Je näher sich der Anteil an dem mindestens einen Leitfähigkeitsadditiv an der genannten Untergrenze bewegt, desto höher ist vorzugsweise der Anteil an dem partikulären Metalloxid. Umgekehrt gilt das Gleiche.

Innerhalb des oben für das partikuläre Metalloxid genannten Bereiches ist ein Anteil im Bereich von 50 Gew.-% bis 90 Gew.-% weiter bevorzugt.

Innerhalb des oben für das mindestens eine Leitfähigkeitsadditiv genannten Bereiches ist ein Anteil im Bereich von 2,5 Gew.-% bis 35 Gew.-% weiter bevorzugt.

Weiterhin bedingen sich vorzugsweise auch die Größe des Überlagerungsbereichs A und der Gesamtanteil G des partikulären Metalloxids und des mindestens einen Leitfähigkeitsadditivs in der Kathode. Je näher sich die Größe des Überlagerungsbereichs an der genannten Untergrenze bewegt, desto höher ist vorzugsweise der Gesamtanteil G. Umgekehrt gilt das Gleiche.

In bevorzugten Weiterbildungen zeichnet sich das Energiespeicherelement durch mindestens eines der unmittelbar folgenden zusätzlichen Merkmale a. bis c. aus:

a. Der Gesamtanteil G des partikulären Metalloxids und des mindestens einen Leitfähigkeitsadditivs in der Kathode liegt im Bereich des 0,85- bis 0,95-fachen des Gesamtgewichts der festen Bestandteile der Kathode.
b. Das Verhältnis des Gewichtsanteils des partikulären Metalloxids zum Gewichtsanteil des mindestens einen Leitfähigkeitsadditivs in der Kathode liegt im Bereich von 20:1 bis 5:1.
c. Für das Verhältnis der Größe des Überlagerungsbereichs A [cm$^2$] zum Gesamtanteils G gilt die folgende Bedingung:

$$x \, [cm^2] * 0{,}95 \le A \, [cm^2] * G$$

In besonders bevorzugter Weise sind die unmittelbar vorstehenden Merkmale a. bis c. in Kombination miteinander realisiert.

Ein hoher Anteil des Metalloxids in der Kathode erhöht die Kapazität des Energiespeicherelements. Für die Strombelastbarkeit ist der Anteil des mindestens einen Leitfähigkeitsadditivs jedoch von grö-ßerer Bedeutung als der Gesamtanteil des Metalloxids.

Betreffend die Wahl eines geeigneten Leitfähigkeitsadditivs sind besonderes die folgenden zwei besonders Varianten bevorzugt.

In der ersten besonders bevorzugten Variante zeichnet sich das Energiespeicherelement durch mindestens eines der unmittelbar folgenden zusätzlichen Merkmale a. und b. aus:

> a. Die Kathode enthält als Leitfähigkeitsadditiv mindestens ein kohlenstoffbasiertes Material, insbesondere aus der Gruppe mit Aktivkohle, Aktivkohlefaser, Carbid-abgeleiteter Kohlenstoff, Kohlenstoff-Aerogel, Graphit, Graphen und Kohlenstoffnanoröhrchen (CNTs).
> b. Die Kathode enthält das mindestens eine kohlenstoffbasierte Material in einem Anteil im Bereich von 25 Gew.-% bis 35 Gew.-% (s.o.).

In besonders bevorzugten Ausführungsformen sind die unmittelbar vorstehenden Merkmale a. und b. in Kombination miteinander realisiert.

In dieser Variante macht man sich zu Nutzen, dass die angegebenen Leitfähigkeitsadditive nicht nur die elektrische Leitfähigkeit der Kathode erhöhen, vielmehr können sie der Kathode zusätzlich zu ihrer Faradayschen Kapazität eine Doppelschichtkapazität verleihen. Somit können für kurze Zeiträume sehr große Ströme zur Verfügung gestellt werden.

Überraschenderweise hat sich herausgestellt, dass sich ein ähnlich positiver Effekt erzielen lässt, indem der Kathode bei ihrer Herstellung über die Elektrodenpaste Leitsalze zugefügt werden, die beim Trocknen der Kathode in der Kathode kristallisieren können. Bei einem späteren Aufdrucken des Elektrolyten können die kristallinen Leitsalze sehr schnell benetzt werden und führen zu einer besseren Durchdringung der Elektroden mit einer ionenleitenden Flüssigkeit.

Entsprechend zeichnet sich das Energiespeicherelement in einer zweiten besonders bevorzugten Variante durch mindestens eines der unmittelbar folgenden zusätzlichen Merkmale a. und b. aus:

> a. Die Kathode enthält als Leitfähigkeitsadditiv mindestens ein wasserlösliches Salz.
> b. Die Kathode enthält das mindestens eine wasserlösliche Salz in einem Anteil im Bereich von 1 Gew.-% bis 25 Gew.-%.

In besonders bevorzugten Ausführungsformen sind die unmittelbar vorstehenden Merkmale a. und b. in Kombination miteinander realisiert.

Als wasserlösliches Salz können der Kathode zum Zwecke der Verbesserung der elektrischen Leitfähigkeit insbesondere Halogenide zugesetzt werden, bevorzugt Chloride, insbesondere Zinkchlorid und/oderAmmoniumchlorid.

In einigen bevorzugten Ausführungsformen können die angeführten Varianten auch kombiniert werden. In diesen Fällen zeichnet sich das Energiespeicherelement durch eine Kombination der unmittelbar folgenden Merkmale a. und b. aus:

> a. Die Kathode enthält als erstes Leitfähigkeitsadditiv das mindestens eine kohlenstoffbasierte Material, insbesondere aus der Gruppe mit Aktivkohle, Aktivkohlefaser, Carbid-abgeleiteter Kohlenstoff, Kohlenstoff-Aerogel, Graphit, Graphen und Kohlenstoffnanoröhrchen (CNTs).
> b. Die Kathode enthält als zweites Leitfähigkeitsadditiv das mindestens eine wasserlösliche Salz.

In bevorzugten Ausführungsformen zeichnet sich das Energiespeicherelement durch eines der unmittelbarfolgenden Merkmale a. und b. aus:

> a. Die Kathode enthält als partikuläres Metalloxid Manganoxid.
> b. Die Kathode enthält als partikuläres Metalloxid Silberoxid.

Bei dem Energiespeicherelement des diagnostischen Pflasters handelt es sich also in besonders bevorzugter Weise um eine Zink/Manganoxid- oder um eine Zink/Silberoxid-Zelle.

In weiteren bevorzugten Ausführungsformen zeichnet sich das Energiespeicherelement durch mindestens eines der unmittelbar folgenden zusätzlichen Merkmale a. bis c. aus:

> a. Die Anode enthält als erstes elastisches Bindemittel oder Bindemittelgemisch mindestens ein Mitglied aus der Gruppe mit Cellulose und deren Derivate, insbesondere Carboxymethylzellulose (CMC), Polyacrylate (PA), Polyacrylsäure (PAA), Polychlortrifluorethylen (PCTFE), Polyhexafluorpropylen (PHFP), Polyimide (PI), Polytetrafluorethylen (PTFE), Polytrifluorethylen (PTrFE), Polyvinylalkohol (PVA), Polyvinylidendifluorid (PVDF), Styrol-Butadien-Gummi (SBR) und Mischungen aus den vorgenannten Materialien.
> b. Die Kathode enthält als zweites elastisches Bindemittel oder Bindemittelgemisch mindestens ein Mitglied aus der Gruppe mit Cellulose und deren Derivate, insbesondere Carboxymethylzellulose (CMC), Polyacrylate (PA), Polyacrylsäure (PAA), Polychlortrifluorethylen (PCTFE), Polyhexafluorpropylen (PHFP), Polyimide (PI), Polytetrafluorethylen (PTFE), Polytrifluorethylen (PTrFE), Polyvinylalkohol (PVA), Polyvinylidendifluorid (PVDF), Styrol-Butadien-Gummi (SBR) und Mischungen aus den vorgenannten Materialien.
> c. Das erste und das zweite elastische Bindemittel oder Bindemittelgemisch sind stofflich identisch.

In besonders bevorzugten Ausführungsformen sind die unmittelbar vorstehenden Merkmale a. und b. in Kombination miteinander realisiert. In einigen Weiterbildungen dieser besonders bevorzugten Ausführungsformen sind die unmittelbar vorstehenden Merkmale a. bis c. in Kombination miteinander realisiert.

Besonders bevorzugt sind sowohl in der Kathode als auch in der Anode als Bindemittel oder Bindemittelgemisch eine Kombination aus einem als Elektrodenbinder geeigneten Zellulosederivat und SBR enthalten. Beispielsweise können die Anode und die Kathode 0,5 Gew.-% bis 2,5 Gew.-% Carboxymethylzellulose und 5 Gew.-% bis 10 Gew.-% SBR enthalten.

Die Anode enthält das erste elastische Bindemittel oder Bindemittelgemisch, bezogen auf das Gesamtgewicht ihrer festen Bestandteile, vorzugsweise in einem Anteil von 1 Gew.-% bis 25 Gew.-%.

Das partikuläre metallische Zink oder die partikuläre metallische Zinklegierung ist in der Anode bevorzugt in einem Anteil im Bereich von 40 Gew.-% bis 80 Gew.-% enthalten.

Gegebenenfalls kann auch die Anode einen Anteil an einem Leitfähigkeitsadditiv enthalten. Da das Aktivmaterial der Anode allerdings bereits von sich aus elektrisch leitfähig ist, ist das nicht zwingend erforderlich.

In einer besonders bevorzugten Ausführungsform des diagnostischen Pflasters ist das Energiespeicherelement durch eine Kombination der vier unmittelbar folgenden zusätzlichen Merkmale a. bis d. gekennzeichnet:

a. Die Kathode enthält als partikuläres Metalloxid ein Metalloxid aus der Gruppe mit Manganoxid und Silberoxid.

b. Die Kathode enthält als Leitfähigkeitsadditiv mindestens ein wasserlösliches Salz.

c. Die Kathode enthält das mindestens eine wasserlösliche Salz in einem Anteil im Bereich von 1 Gew.-% bis 25 Gew.-%.

d. Die Kathode enthält als zweites elastisches Bindemittel oder Bindemittelgemisch eine Kombination aus Carboxymethylzellulose und SBR.

Es wurde eingangs bereits erwähnt, dass in einigen Ausführungsformen auch der Separator gedruckt werden kann. Geeignete Druckpasten hierfür sind beispielsweise der EP 2 561564 B1 zu entnehmen.

In weiteren Ausführungsformen kann es sich bei dem Separator aber auch um ein poröses Flächengebilde, beispielsweise eine poröse Folie oder ein Vlies, handeln, das zwischen der Anode und der Kathode angeordnet wird. Geeignete Flächengebilde und entsprechende Vorgehensweisen sind in der EP 3 477 727 A1 beschrieben.

Nicht zuletzt ist es möglich, dass zwischen den Elektrolyten ein Festelektrolyt angeordnet wird, wie er in einer bevorzugten Ausführungsform beispielsweise in der EP 2 960 967 B1 beschrieben ist, wobei Varianten, bei denen ein flüssiger Elektrolyt zum Einsatz kommen, in vielen Fällen aber bevorzugt sind.

Entsprechend kann sich das Energiespeicherelement des diagnostischen Pflasters bevorzugt durch mindestens eines der unmittelbar folgenden zusätzlichen Merkmale a. und b. auszeichnen:

a. Es umfasst einen wässrigen Elektrolyten, der ein chloridbasiertes Leitsalz enthält,

b. Der Separator, der zwischen der Anode und der Kathode angeordnet ist, ist mit dem Elektrolyten getränkt.

In besonders bevorzugten Ausführungsformen sind die unmittelbar vorstehenden Merkmale a. und b. in Kombination miteinander realisiert.

Als chloridbasiertes Leitsalz sind insbesondere Zinkchlorid und Ammoniumchlorid geeignet. Es ist bevorzugt, dass sich der pH-Wert des wässrigen Elektrolyten im neutralen oder leicht sauren Bereich bewegt.

Wie oben erwähnt, stehen die Elektroden des Energiespeicherelements jeweils in unmittelbarem Kontakt mit einem elektrisch leitenden Stromleiter, der die elektrische Anbindung des Energiespeicherelements an den jeweiligen Verbraucher gewährleistet. Bei diesen elektrischen Leitern haandelt es sich bevorzugt um Leiterbahnen aus Metallpartikeln, beispielsweise aus Silberpartikeln oder aus Partikeln aus einer Silberlegierung. Druckbare Leitpasten zum Beispiel mit Silberpartikeln zur Herstellung der elektrischen Leiter sind bekannt und im Handel frei erhältlich.

Zink-Luft-Zelle

In anderen besonders bevorzugten Ausgestaltungen des erfindungsgemäßen diagnostischen Pflasters handelt es sich bei dem Energiespeicherelement nicht um eine in den vorherigen Abschnitten beschriebene Zink-Metalloxid-Zelle, sondern um eine Zink-Luft-Zelle. Das Energiespeicherelement ist dann bevorzugt durch die folgenden zusätzlichen Merkmale gekennzeichnet:

a. die negative Elektrode umfasst ein erstes elastisches Bindemittel oder Bindemittelgemisch,
b. die positive Elektrode umfasst ein zweites elastisches Bindemittel oder Bindemittelgemisch,
c. der Katalysator, der eine Reduktion von Luftsauerstoff ermöglicht, ist Manganoxid und/oder ein Edelmetall,
d. die positive Elektrode umfasst mindestens ein Leitfähigkeitsadditiv, insbesondere ein Kohlenstoffbasiertes Leitfähigkeitsadditiv,

e. der wässrige Elektrolyt ist ein alkalischer Elektrolyt.

Bei Zink-Luft-Zellen handelt es um meist nicht wiederaufladbare Energiespeicherelemente, die sich durch eine relativ hohe Energiedichte auszeichnen. Als Aktivmaterial für die negative Elektrode kommen meist Partikel aus metallischem Zink oder aus einer metallischen Zinklegierung zum Einsatz. Der Katalysator in der positiven Elektrode dient dazu, bei der Entladung des Energiespeicherelements Sauerstoff unter Elektronenaufnahme zu reduzieren. Die positive Elektrode wird daher oft auch als Sauerstoffkathode bezeichnet. Es entstehen hierbei in der Regel Hydroxidionen, die über den Elektrolyten zur Anode bzw. zur negativen Elektrode wandern können. Dort wird das enthaltene Zink unter Elektronenabgabe oxidiert und die entstehenden Metallionen können mit den Hydroxidionen abreagieren. Zusammengefasst wird also Zink oder gegebenenfalls ein anderes Metall mit Luftsauerstoff in einem vorzugsweise alkalischen Elektrolyten zu Oxid oder Hydroxid oxidiert. Die dabei frei werdende Energie kann elektrochemisch genutzt werden.

Wichtig hierbei ist, dass für die Reaktion Luftsauerstoff zur Verfügung steht. Hierfür ist es zweckmäßigerweise vorgesehen, dass das erfindungsgemäße diagnostische Pflaster im Bereich des Energiespeicherelements eine Durchlässigkeit für Luftsauerstoff ermöglicht. Hierfür können beispielsweise eine oder gegebenenfalls mehrere Luftzuführöffnungen im Bereich des Energiespeicherelements vorgesehen sein oder eine allgemein luftdurchlässige Abdeckung bzw. ein luftdurchlässiges Gehäuse für das Energiespeicherelement vorgesehen sein.

In besonders bevorzugter Weise können ein oder mehrere Luftzuführöffnungen vorgesehen sein, die vor dem Gebrauch des diagnostischen Pflasters beispielsweise mit einer Klebefolie abgedeckt oder anderweitig versiegelt sind. Bei Ingebrauchnahme des diagnostischen Pflasters können das oder die Siegel entfernt werden, so dass erst dann die Redoxreaktionen in dem Energiespeicherelement und damit die Bereitstellung der Energie einsetzen kann.

Das Energiespeicherelement ist hinsichtlich des ersten und des zweiten elastischen Bindemittels oder Bindemittelgemisches sowie hinsichtlich des mindestens einen Leitfähigkeitsadditivs bevorzugt ausgestaltet wie im Falle der Zink-Metalloxid-Zelle.

Bei der Sauerstoffkathode kann es sich in bevorzugten Ausgestaltungen um eine kunststoffgebundene Gasdiffusionselektrode handeln. Hierbei bildet ein Kunststoffbinder, beispielsweise Polytetrafluorethylen (PTFE), eine poröse Matrix aus, in die als Katalysator Partikel aus einem elektrokatalytisch aktiven Material eingelagert sind. Bei diesem elektrokatalytisch aktiven Material bzw. dem Katalysator handelt es sich vorzugsweise um Manganoxid und/oder ein Edelmetall, wie zum Beispiel Platin oder Palladium.

Bei dem Kohlenstoff-basierten Leitfähigkeitsadditiv handelt es sich beispielsweise um Ruß und/oder Graphit und/oder Aktivkohle. Die beispielsweise von einem Kunststoffbinder gebildete poröse Matrix der Gasdiffusionselektrode kann beispielsweise mit einer Schicht aus feinteiligem Kohlenstoff, die mit der Katalysatorsubstanz versetzt ist, beschichtet sein. Diese Aktivschicht kann beispielsweise eine Dicke von etwa 1 mm haben.

Bei dem alkalischen Elektrolyten kann es sich insbesondere um eine Lauge handeln, beispielsweise um Kalilauge. Vorzugsweise ist die Sauerstoffkathode mit dem Elektrolyten benetzt.

Beschreibung der Einrichtung zur drahtlosen Datenübertragung

In Bezug auf die Einrichtung zur drahtlosen Datenübertragung des erfindungsgemäßen diagnostischen Pflasters ist das diagnostische Pflaster in bevorzugten Ausgestaltungen durch mindestens eines der unmittelbar folgenden zusätzlichen Merkmale a. bis d. gekennzeichnet:

     a. die Einrichtung zur drahtlosen Datenübertragung basiert auf einem NFC-Übertragungsstandard,
     b. die Einrichtung zur drahtlosen Datenübertragung basiert auf einem Mobilfunkstandard, insbesondere auf einem LTE-Standard,
     c. die Einrichtung zur drahtlosen Datenübertragung basiert auf einem Wireless Personal Area Network (WPAN) Standard, insbesondere einem Bluetooth-Standard,
     d. die Einrichtung ist zur drahtlosen Übertragung von Daten über eine Reichweite von 5 cm bis 20 m, insbesondere von 10 cm bis 10 m, eingerichtet.

Die drahtlose Datenübertragung bei dem erfindungsgemäßen diagnostischen Pflaster kann prinzipiell auf verschiedene Weise und anhand von dem Fachmann geläufigen Datenübertragungsstandards erfolgen.

Je nach konkreter Anwendung kann mit der drahtlosen Datenübertragung eine relativ kurze Strecke, beispielsweise im Zentimeterbereich, oder eine längere Strecke, beispielsweise bis zu 20 m, überwunden werden. Beispielsweise kann eine Datenübertragung zu einem mobilen Endgerät, wie beispielsweise einem Smartphone, vorgesehen sein, das der Proband oder ein anderer Nutzer bei sich trägt. In solchen Fällen muss nur eine verhältnismäßig kurze Distanz überwunden werden, so dass die drahtlose Datenübertragung beispielsweise für eine Reichweite in einem Bereich zwischen 5 cm und 100 cm für diese Anwendung ausreichend ist. Bei anderen Anwendungen kann eine größere Reichweite sinnvoll sein, beispielsweise wenn die Daten an ein spezielles Auswertegerät im Raum, beispielsweise an einen Computer oder anderes, übertragen werden sollen. In solchen Fällen können Reichweiten bis zu beispielsweise 20 m oder bis zu beispielsweise 10 m sinnvoll sein.

Für verhältnismäßig kurze Reichweiten kann beispielsweise auf eine Datenübertragung gemäß dem dem Fachmann bekannten Bluetooth-Standard zurückgegriffen werden. Ebenfalls geeignet für eine Datenübertragung über verhältnismäßig kurze Strecken ist der Einsatz des NFC (*Near Field Commu-nication*)-Übertragungsstandards, bei dem per elektromagnetischer Induktion mittels loser gekoppelter Spulen beispielsweise über einige Zentimeter eine Datenübertragung möglich ist.

Für eine drahtlose Datenübertragung über längere Strecken bzw. mit einer größeren Reichweite eignen sich in erster Linie Mobilfunkstandards, insbesondere LTE (*Long Term Evolution*)-Standards. Die für eine solche drahtlose Datenübertragung über längere Strecken erforderliche erhöhte Energiedichte ist mit besonderem Vorteil mittels der beschriebenen Zink-Metalloxid-Zellen als Energiespeicherelemente bei dem diagnostischen Pflaster möglich, da mit derartigen Energiespeicherelementen elektrische Ströme in der Größenordnung von 400 mA oder mehr bereitgestellt werden kann. Auch die Zink-Luft-Zellen als Energiespeicherelemente eignen sich hierfür.

Je nach Anwendung, z. B. im Bereich der Telemedizin, kann auch eine noch größere Reichweite vorgesehen sein, beispielsweise durch eine Kombination mit GPS (*Global Positioning System)*-Standards, die gegebenenfalls eine Übertragung auch über Kilometer hinweg ermöglichen.

Insgesamt bietet das erfindungsgemäße diagnostische Pflaster für den Probanden einen hohen Tragekomfort und eine freie Beweglichkeit durch die drahtlose Datenübertragung. Beispielsweise kann das diagnostische Pflaster am Körperstamm angebracht werden. Durch entsprechende Ausgestaltung der sensorischen Einrichtung des diagnostischen Pflasters ist prinzipiell eine kontinuierliche Überwachung verschiedener Körperfunktionen ohne eine Bewegungseinschränkung für den Probanden möglich. Auch ein versehentliches Abstreifen der Sensorik wird vermieden, so dass insgesamt das erfindungsgemäße diagnostische Pflaster mit einer hohen Akzeptanz bei dem Probanden verbunden ist.

In Bezug auf die Sensorik-Einrichtung des erfindungsgemäßen diagnostischen Pflasters ist das diagnostische Pflaster in bevorzugten Ausgestaltungen mit mindestens einem der unmittelbar folgenden zusätzlichen Merkmale a. bis c. gekennzeichnet:

    a. die Sensorik-Einrichtung umfasst eine optische Sensorik,
    b. die Sensorik-Einrichtung umfasst eine Spannungsmess-Sensorik,
    c. die Sensorik-Einrichtung umfasst mindestens eine Temperatursensorik.

Prinzipiell ist die Sensorik-Einrichtung des erfindungsgemäßen diagnostischen Pflasters nicht auf eine bestimmte Sensorik eingeschränkt, sondern kann vielmehr an unterschiedliche sensorische Bedürfnisse angepasst und für die Überwachung und/oder Analyse unterschiedlichster Körperfunktionen und/oder Körpermerkmale eingesetzt werden. Besonders bevorzugt ist eine Kombination der vorgenannten Merkmale a. und b. oder b. und c.

Bei der Temperatursensorik kann es sich insbesondere um eine optische Sensorik, z.B. um einen Infrarotsensor, handeln. Die Sensorik kann aber auch ein Thermoelement oder einen elektronischen Temperatursensor, der in Abhängigkeit der Temperatur seinen Widerstand ändert, umfassen.

Für viele geeignete Anwendung kann eine optische Sensorik zum Einsatz kommen, da sehr viele, bereits etablierte diagnostische Methoden auf einer optischen Sensorik beruhen. Eine Spannungsmess-Sensorik, insbesondere im Hinblick auf elektrische Aktivitäten der Herzmuskelfasern, und/oder eine Temperatursensorik, insbesondere zur Überwachung der Körpertemperatur, können alternativ und in bevorzugter Weise auch zusätzlich bei dem diagnostischen Pflaster vorgesehen sein.

Die optische Sensorik des erfindungsgemäßen diagnostischen Pflasters zeichnet sich in besonders bevorzugten Ausgestaltungen insbesondere durch mindestens eines der unmittelbar folgenden zusätzlichen Merkmale a. bis k. aus:

    a. die optische Sensorik ist zur Analyse von Blutgaswerten eingerichtet,
    b. die optische Sensorik ist zur Analyse der peripheren kapillaren Sauerstoffsättigung eingerichtet,
    c. die optische Sensorik ist zur Pulsmessung eingerichtet,
    d. die optische Sensorik ist zur Pulsoxymetrie eingerichtet,
    e. die optische Sensorik ist zur Erfassung der Herzfrequenz eingerichtet,
    f. die optische Sensorik ist zur Analyse der Kohlenstoffmonoxid-Beladung im Blut eingerichtet,
    g. die optische Sensorik ist zur Analyse des $CO_2$-Gehalts und/oder des $HCO_3$-Gehaks im Blut eingerichtet,
    h. die optische Sensorik ist zur Analyse des Gewebezuckergehaltes eingerichtet,
    i. die optische Sensorik ist zur Analyse einer Metallionenkonzentration in Körperflüssigkeiten eingerichtet,
    j. die optische Sensorik ist zur Analyse einer Laktatkonzentration in Körperflüssigkeiten eingerichtet,
    k. die optische Sensorik ist zur Temperaturmessung eingerichtet.

Im medizinischen Rettungsdienst, in der Klinik, in Pflegeheimen, aber auch im häuslichen Gebrauch hat sich in vielen Bereichen eine Überwachung verschiedener Körperfunktionen etabliert. Oftmals geht es hierbei um eine Überwachung der Sauerstoffsättigung im Blut. Neben dem Sauerstoff spielen auch andere Blutgaswerte eine große Rolle in der Diagnostik, beispielsweise kann aus dem $CO_2$-Gehalt und dem $HCO_3$-Gehalt der pH-Wert des Bluts errechnet werden, der eine wichtige diagnostische Größe darstellt. Aber auch andere Parameter, insbesondere die Herzfrequenz, werden vielfach überwacht.

Insbesondere bei Notfalleinsätzen mit Brand- und/oder Rauchentwicklung spielt eine CO-Intoxikation eine große Rolle, so dass eine Analyse der Kohlenstoffmonoxid-Beladung im Blut eine wichtige diagnostische Größe ist, um gegebenenfalls dringend erforderliche therapeutische Maßnahmen einleiten zu können.

Eine weitere, besonders wichtige diagnostische Größe insbesondere im Hinblick auf eine Diabetes-Erkrankung stellt der Blutzuckergehalt dar, der mithilfe einer geeigneten optischen Sensorik indirekt anhand einer Analyse des Gewebezuckergehalts erfasst werden kann.

Weiterhin spielen Metallionenkonzentrationen in Körperflüssigkeiten, insbesondere der Kalium ($K^+$)-Gehalt, als diagnostische Größe eine Rolle. Auch andere, im Blut enthaltene Elektrolyte, beispielsweise Natrium und/oder Magnesium, können von Interesse sein.

Darüber hinaus kann die Laktatkonzentration in Körperflüssigkeiten für verschiedene Fragestellungen ebenfalls eine wichtige diagnostische Größe darstellen.

Bisher wird herkömmlicherweise die Sauerstoffsättigung im Blut bzw. die periphere kapillare Sauerstoffsättigung anhand eines Sensors, der in der Regel am Finger oder gegebenenfalls auch am Zeh oder am Ohr angebracht wird, im Durchlichtverfahren gemessen. Dieser Sensor ist über Kabel mit einer entsprechenden Auswerteeinheit bzw. Anzeigeeinheit verbunden. Die Messung basiert dabei prinzipiell darauf, dass Licht in das Gewebe eingestrahlt wird. Da sich das Absorptionsverhalten von sauerstoffreichem Blut und sauerstoffarmem Blut voneinander unterscheidet, kann durch Analyse des reflektierten Lichts bzw. des austretenden Lichts ein Rückschluss auf die Sauerstoffsättigung des Bluts gezogen werden.

Die bei herkömmlichen Vorrichtungen zur Messung der Sauerstoffsättigung oftmals störenden erforderlichen Kabel werden bei dem erfindungsgemäßen diagnostischen Pflaster vermieden, da bei dem erfindungsgemäßen diagnostischen Pflaster eine drahtlose Datenübertragung an ein externes Anzeige- und/oder Auswertegerät vorgesehen ist. Zudem stellt die Anbringung der hierfür erforderlichen optischen Sensorik in Form eines Pflasters für den Probanden nur eine sehr geringe Belastung und Einschränkung dar, so dass diese Überwachung im Vergleich mit herkömmlichen Vorrichtungen als weit weniger belastend empfunden wird. Hierzu trägt auch der Verzicht auf eine Verkabelung bei dem erfindungsgemäßen diagnostischen Pflaster erheblich bei.

Die Messung der Sauerstoffsättigung mittels des erfindungsgemäßen diagnostischen Pflasters kann beispielsweise auf einem Durchlichtverfahren beruhen, wie es bereits für herkömmliche "Sensorclips" eingesetzt wird, die am Finger, Zeh oder Ohrläppchen eines Patienten/Probanden angebracht werden. In besonders bevorzugter Weise basiert die Messung der Sauerstoffsättigung bei dem erfindungsgemäßen diagnostischen Pflaster jedoch auf einer Analyse des reflektierten Lichts, so dass das diagnostische Pflaster auch in der Fläche, beispielsweise am Körperstamm, eingesetzt werden kann.

Die mit dem erfindungsgemäßen diagnostischen Pflaster durch entsprechende Einrichtung der Sensorik mögliche Analyse verschiedener Blutwerte ist im Vergleich mit einer üblichen Blutwertanalyse, die in der Regel mit einer Blutabnahme verbunden ist, durch ihren nicht-invasiven Charakter für den Probanden wesentlich angenehmer.

Je nach konkreter Ausgestaltung kann die optische Sensorik des diagnostischen Pflasters im Hinblick auf verschiedene Analysemöglichkeiten ausgestaltet sein. Insbesondere können verschiedene diagnostische Größen in kombinierter Weise erfasst werden. Beispielsweise kann die optische Sensorik für eine Pulsoxymetrie, also eine kombinierte Analyse der peripheren kapillaren Sauerstoffsättigung und einer Pulsmessung, eingerichtet sein. Mit besonderem Vorteil kann durch entsprechende Einrichtung der optischen Sensorik sowohl eine Analyse der Kohlenstoffmonoxid-Beladung als auch eine Messung der peripheren kapillaren Sauerstoffsättigung möglich sein, wodurch das erfindungsgemäße diagnostische Pflaster unter anderem mit besonderem Vorteil in der Rettungsmedizin und bei Notfalleinsätzen einsetzbar ist.

In besonders bevorzugter Weise ist die optische Sensorik des erfindungsgemäßen diagnostischen Pflasters durch die folgenden zusätzlichen Merkmale a. und b. gekennzeichnet:

a. die optische Sensorik weist mindestens eine Lichtwellen-emittierende Einheit auf,
b. die optische Sensorik weist mindestens eine Einheit zum Empfangen von Lichtwellen und zur Umwandlung der empfangenen Lichtwellen in ein elektrisches Signal auf.

Zweckmäßigerweise sind die vorgenannten Merkmale a. und b. in Kombination miteinander verwirklicht. Mit einer solchen Ausgestaltung der optischen Sensorik kann insbesondere die Konzentration von Analyten in einer Körperflüssigkeit untersucht werden, die sich durch ein bestimmtes Absorptionsverhalten von elektromagnetischer Strahlung bestimmter Wellenlänge auszeichnen.

In besonders bevorzugter Weise zeichnet sich die optische Sensorik des erfindungsgemäßen diagnostischen Pflasters weiterhin durch mindestens eines der unmittelbar folgenden zusätzlichen Merkmale a. bis e. aus:

> a. die optische Sensorik weist zwei Lichtwellen-emittierende Einheiten auf, wobei die einzelnen Lichtwellen-emittierenden Einheiten Lichtwellen mit unterschiedlicher Wellenlänge emittieren,
> b. die optische Sensorik weist drei Lichtwellen-emittierende Einheiten auf, wobei die einzelnen Lichtwellen-emittierenden Einheiten Lichtwellen mit unterschiedlicher Wellenlänge emittieren,
> c. die optische Sensorik weist vier bis sieben Lichtwellen-emittierende Einheiten auf, wobei die einzelnen Lichtwellen-emittierenden Einheiten Lichtwellen mit unterschiedlicher Wellenlänge emittieren,
> d. die Lichtwellen-emittierende Einheit ist zur Emission von Laserstrahlen eingerichtet,
> e. die Lichtwellen-emittierende Einheit ist zur Emission von Fluoreszenzstrahlen eingerichtet.

Für die Analyse der Sauerstoffsättigung werden üblicherweise zwei oder drei unterschiedliche Wellenlängen im Gewebe gemessen. Diese Wellenlängen können sich im Bereich des sichtbaren Lichts befinden oder aber auch im Infrarot-Bereich oder Ultraviolett (UV)-Bereich. Besonders bevorzugt für das erfindungsgemäße diagnostische Pflaster ist die Verwendung von Lichtwellen mit drei unterschiedlichen Wellenlängen.

In anderen Ausgestaltungen des diagnostischen Pflasters können vier bis sieben unterschiedliche Wellenlängen verwendet werden. Dies hat den Vorteil, dass zwischen $O_2$ und CO, die am Hämoglobin gebunden sein können, unterschieden werden kann. Mit einer solchen Ausgestaltung des diagnostischen Pflasters kann also insbesondere erkannt werden, ob eine Kohlenmonoxidvergiftung vorliegt. Durch entsprechende Auswahl und Anpassung der Lichtwellen-emittierenden Einheiten können auch andere Blutgaswerte, insbesondere $CO_2$ und $HCO_3$, analysiert werden, wobei aus diesen Größen insbesondere auch der pH-Wert des Bluts abgeleitet werden kann.

Für eine Anwendung im Rettungsdienst kann beispielsweise ein erfindungsgemäßes diagnostisches Pflaster mit einer Sensorik für eine Pulsoxymetrie direkt am Körperstamm des Patienten aufgeklebt werden, so dass eine gesicherte und genaue Messung der Sauerstoffsättigung im Blut und des Pulses möglich ist. Insbesondere bei Verdacht auf eine Kohlenstoffmonoxidvergiftung kann ein diagnostisches Pflaster mit einer gewissermaßen erweiterten optischen Sensorik für eine Messung mit vier bis sieben verschiedenen Wellenlängen eingesetzt werden, um eine genaue Analyse der Blutgaswerte zu ermöglichen.

In anderen Ausgestaltungen kann die optische Sensorik so ausgerichtet sein, dass die Lichtwellen-emittierende Einheit zu Emissionen von Laserstrahlen eingerichtet ist. Die Verwendung von Laserstrahlen ist insbesondere im Hinblick auf eine Glukosemessung bzw.

Zuckermessung in Körperflüssigkeiten besonders geeignet. Eine Ausgestaltung der Sensorik mit Laserstrahlen kann daher beispielsweise für eine nicht-invasive Blutzuckermessung anhand einer Analyse der auf der Haut vorkommenden Feuchtigkeiten vorgenommen werden. In anderen Ausgestaltungen kann mit dem erfindungsgemäßen diagnostischen Pflaster eine Glukosemessung im Unterhautfettgewebe erfolgen, wobei für diese Ausgestaltungen die Einbringung einer dünnen Nadel in das Unterhautfettgewebe zur Bereitstellung von Proben der Gewebsflüssigkeit erforderlich ist.

In anderen Ausgestaltungen können Fluoreszenzstrahlen eingesetzt werden, die für verschiedene diagnostische Messgrößen mit Vorteil genutzt werden können. Beispielsweise kann mit einer Fluoreszenzmessung eine Kalium-Bestimmung in Körperschweiß, Gewebeflüssigkeit oder anderen Körperflüssigkeiten erfolgen. In ähnlicher Weise kann prinzipiell eine Laktatbestimmung erfolgen, wie es sich für einen Fachmann auf diesem Gebiet erschließt.

Die oben erwähnte mögliche Spannungsmess-Sensorik des erfindungsgemäßen diagnostischen Pflasters ist insbesondere zur Erfassung eines Elektrokardiogramms (EKG) eingerichtet, wobei die elektrischen Aktivitäten der Herzmuskelfasern erfasst werden. Da jeder Kontraktion des Herzmuskels eine elektrische Erregung vorausgeht, sind die elektrischen Spannungsänderungen am Herzen an der Körperoberfläche messbar. Die für diese Spannungsmessung erforderlichen Sensoren werden üblicherweise an verschiedenen, vorgegebenen Körperpositionen angebracht. Herkömmlicherweise werden in der Regel sogenannte 3-er-EKGs oder 12-er-EKGs aufgenommen, die entweder drei oder vier (im Falle des 3-er EKGs) oder zehn Sensoren (im Falle des 12-er EKGs) an definierten Körperpositionen verwenden. Insbesondere bei einem 12-er EKG können dabei auch Spannungsunterschiede zwischen einzelnen Sensoren ausgewertet werden. Bei der Auswertung der Messdaten ist es in der Regel erforderlich, dass die entsprechenden Positionen der Sensoren am Körper des Probanden berücksichtigt werden. Insbesondere für diese Anwendung des erfindungsgemäßen diagnostischen Pflasters ist das Pflaster zweckmäßigerweise auch mit einer entsprechenden Informationsverarbeitungsmöglichkeit ausgestattet.

Gegenüber herkömmlichen Sensoren für die Aufnahme von Messdaten für ein Elektrokardiogramm hat das erfindungsgemäße diagnostische Pflaster auch in diesem Zusammenhang den besonderen Vorteil, dass auf eine störende Verkabelung verzichtet wird. Darüber hinaus können hierdurch insbesondere in der Notfallmedizin störenden Mess-Artefakten, die bei größeren Bewegungen bzw. bei dem Transport des Patienten entstehen, vermieden werden. Auch bei der Erstellung von sogenannten 24-Stunden-EKGs, die beispielsweise zur Abklärung von beginnenden Herzkrankheiten oder unklaren Krankheitsbildern vorgenommen werden, stellt der Verzicht auf eine Verkabelung einen erheblichen Anwendungsvorteil dar, da der Proband nicht mit störenden und hinderlichen Kabel konfrontiert ist.

Ein besonderer Vorteil des erfindungsgemäßen diagnostischen Pflasters ist, dass die verschiedenen sensorischen Möglichkeiten miteinander kombiniert werden können, beispielsweise die Analyse der Sauerstoffsättigung im Blut und die Erfassung von Sensordaten für ein Elektrokardiogramm.

Auch im intensivmedizinischen Bereich und/oder im Neugeborenen- oder Frühgeborenenbereich von Krankenhäusern können durch die Verwendung der erfindungsgemäßen diagnostischen Pflaster besondere Vorteile erreicht werden, da diese Pflaster eine deutliche Erleichterung für die Patienten und das Personal bieten. Vor allem der Verzicht auf eine Verkabelung erleichtert eine Bewegung und/oder Lagerung der Patienten. Weiterhin wird beispielsweise ein versehentliches Abstreifen der Sensorik vermieden. Ähnliches gilt für die häusliche Krankenpflege oder die Pflege in Senioren- oder Pflegeheimen. Auch für die Überwachung von beispielsweise Asthma- oder COPD-Patienten (COPD - *Chronic Obstructive Pulmonary Disease),* bei denen die Sauerstoffversorgung möglicherweise kritisch ist, erlaubt die Verwendung der erfindungsgemäßen sensorischen Pflaster eine kontinuierliche Überwachung bei nicht weiter eingeschränkter Bewegungsfreiheit für den Patienten. Auch beispielsweise in der Schlafmedizin ermöglichen die erfindungsgemäßen diagnostischen Pflaster eine kontinuierliche Überwachung ohne eine Einschränkung der Bewegungsfreiheit und Bequemlichkeit für den Probanden. Weiterhin kann das erfindungsgemäße diagnostische Pflaster beispielsweise im Operationssaal eines Krankenhauses mit Vorteil zum Einsatz kommen, insbesondere im Hinblick auf eine genaue Überwachung des Patienten während der Narkose.

Die Möglichkeit der Anbringung des oder der diagnostischen Pflaster am Körperstamm oder an anderen flächigen Bereichen des Körpers hat neben der verbesserten Bequemlichkeit für Patient/Proband und behandelndem Personal auch noch weitere Vorteile. Insbesondere im Hinblick auf die Messung der Sauerstoffsättigung im Blut, die herkömmlicherweise durch Messung mittels Fingersensoren (Messung am Finger oder am Zeh) erfolgt, hat die Messung mittels des erfindungsgemäßen diagnostischen Pflasters den Vorteil, dass die Messung nicht zwingend am Finger oder Zeh erfolgen muss, sondern auch am Körperstamm oder an einem anderen flächigen Bereich des Körpers möglich ist. Dies ist insbesondere bei kleinen Kindern oder Säuglingen mit sehr kleinen Fingern und beispielsweise bei Senioren oder anderen Personen, bei denen Hornhaut oder andere Hautveränderungen an den Zehen oder Fingern die Messung erschweren würden, sehr vorteilhaft. Die Messung am Körperstamm mittels des erfindungsgemäßen diagnostischen Pflasters ist auch bei unterkühlten Patienten sehr vorteilhaft, da es bei diesen Patienten zu einer verminderten Durchblutung der Extremitäten kommen kann, wodurch eine Messung am Finger oder Zeh zu einer Verfälschung der Messergebnisse führen würde. Weiterhin ist die Messung am Körperstamm auch in solchen Fällen einer Messung am Finger oder Zeh überlegen, in denen lackierte oder künstliche Finger- oder Zehennägel des Patienten/Probanden die Messung mit herkömmlichen Sensoren erschweren, da herkömmliche Sensoren in der Regel mit einem Durchlichtverfahren arbeiten. Weiterhin bietet das erfindungsgemäße diagnostische Pflaster bei sogenannten "zentralisierten" Patienten durch die Anbringung am Körperstamm besondere Vorteile. Bei zentralisierten Patienten ist ein niedriger bis sehr niedriger Blutdruck zu beobachten, wobei hauptsächlich nur noch die wichtigsten Körperteile mit Blut versorgt werden. Dies bedingt, dass die Extremitäten weniger oder nicht mehr mit Blut versorgt werden, sodass eine Messung der Sauerstoffsättigung am Finger oder Zeh nicht mehr möglich ist. Derartige Zustände können beispielsweise bei einem Schockzustand durch Blut- und/oder Flüssigkeitsverlust, bei akuten Herzproblemen, bei allergischen Reaktionen, bei Sepsis und/oder Vergiftung, bei neurologischen Störungen oder im Fall einer Reanimation auftreten.

In besonders bevorzugter Weise weist das erfindungsgemäße diagnostische Pflaster mindestens eine Abschirmschicht zur Abschirmung von elektromagnetischer Strahlung auf. Dies ist insbesondere im Hinblick auf eine optische Sensorik bei dem diagnostischen Pflaster vorteilhaft, da hierdurch störende Strahlung ausgeschaltet werden kann, die das Messergebnis gegebenenfalls verfälschen kann. Die Abschirmschicht kann in einfacher Weise durch optisch undurchlässige Schichten auf dem Pflaster realisiert werden.

Weiterhin kann das diagnostische Pflaster in einer besonders bevorzugten Ausgestaltung mindestens eine Elektronik-Einheit zur Datenverarbeitung aufweisen. Diese Elektronik-Einheit kann beispielsweise in Form eines Mikrochips auf dem Pflaster angebracht werden. Eine solche Elektronik-Einheit ist insbesondere im Zusammenhang mit der Erfassung eines Elektrokardiogramms sinnvoll, da bei dieser Anwendung die Position des jeweiligen Pflasters am Körper des Probanden definiert bzw. dem Messsystem aufgezeigt werden sollte. Prinzipiell kann auch eine externe Elektronik-Einheit, sofern erforderlich, eingesetzt werden und der entsprechende Dateninput für die Elektronik-Einheit kann über die drahtlose Datenübertragung des diagnostischen Pflasters bewerkstelligt werden.

In besonders bevorzugter Weise ist das diagnostische Pflaster und sind insbesondere die elektronischen Komponenten des diagnostischen Pflasters so ausgestaltet, dass sie auch bei hohen Stromflüssen nicht geschädigt werden. Vorzugsweise ist das diagnostische Pflaster dabei mit geeigneten Stromsicherungen versehen. Der Vorteil dieser Ausführungsform liegt darin, dass während der Verwendung des oder der diagnostischen Pflaster die Notwendigkeit einer Reanimation oder allgemein einer externen Defibrillation des Patienten auftreten kann. Eine Defibrillation kann insbesondere bei einer Herz-Lungen-Wiederbelebung oder bei der Beseitigung und/oder Behandlung von Herzrhythmusstörungen erforderlich sein. Durch entsprechende Stromsicherungsmaßnahmen wird die Elektronik des diagnostischen Pflasters bei den mit einer externen Defibrillation verbundenen hohen Stromstößen nicht geschädigt, sodass das diagnostische Pflaster anschließend wieder mit voller Funktionsfähigkeit weiterverwendet werden kann.

Bei dem flächigen Substrat des erfindungsgemäßen diagnostischen Pflasters kann es sich beispielsweise um einen Schaumstoffträger handeln, der insbesondere mit einer Kleberschicht versehen ist. Mittels der Kleberschicht wird der Schaumstoffträger, der die beschriebenen verschiedenen Einheiten des diagnostischen Pflasters trägt, auf der Körperoberfläche des Probanden befestigt. Vor der Anbringung des Pflasters an dem Körper des Probanden ist das diagnostische Pflaster vorzugsweise mit einer flächigen und entfernbaren Kleberschutzschicht versehen, um die Kleberschicht vor der Anwendung zu schützen. Für die Kleberschicht und den Schaumstoffträger können prinzipiell an sich bekannte Materialien verwendet werden, die dem Fachmann geläufig sind.

Die Erfindung umfasst weiterhin ein Verfahren zur Herstellung eines diagnostischen Pflasters gemäß der obigen Beschreibung. Dieses Verfahren umfasst zumindest die unmittelbar folgenden Schritte:

> a. es wird ein flächiges Substrat bereitgestellt, das zur Aufbringung auf die Körperoberfläche eines Probanden geeignet ist,
> b. auf das flächige Substrat wird mindestens eine Sensorik-Einrichtung aufgebracht,
> c. auf das flächige Substrat wird mindestens eine Einrichtung zur drahtlosen Datenübertragung aufgebracht,
> d. auf das flächige Substrat wird mindestens ein Energiespeicherelement aufgedruckt oder auf dem flächigen Substrat wird ein gedrucktes Energiespeicherelement angeordnet.

In Bezug auf das Drucken des Energiespeicherelements zeichnet sich das Verfahren in bevorzugter Weise weiter durch mindestens einen der unmittelbar folgenden zusätzlichen Schritte a. bis d. aus:

> a. es wird ein erster elektrischer Leiter und ein zweiter elektrischer Leiter gedruckt,
> b. es wird eine negative Elektrode als Schicht auf den ersten elektrischen Leiter unter Verwendung einer Druckpaste, die partikuläres metallisches Zink oder eine partikuläre metallische Zinklegierung enthält, und die ein erstes elastisches Bindemittel oder Bindemittelgemisch enthält, gedruckt,
> c. es wird eine positive Elektrode als Schicht auf den zweiten elektrischen Leiter unter Verwendung einer Druckpaste, die ein partikuläres Metalloxid und/oder einen Katalysator, der eine Reduktion von Luftsauerstoff ermöglicht, und ein zweites elastisches Bindemittel oder Bindemittelgemisch enthält, gedruckt,
> d. es wird die negative Elektrode und die positive Elektrode mit einem flüssigen Elektrolyten getränkt.

Für die Herstellung der negativen Elektrode einer Zink-Metalloxid-Zelle als Energiespeicherelement kann insbesondere eine druckfähige Zinkpaste verwendet werden, die als elektrochemische Aktivkomponente Zinkpulver sowie weiterhin einen geeigneten Binder und ein geeignetes Lösungs- und/oder Dispergiermittel umfasst. Zur Herstellung der positiven Elektrode einer solchen Zelle kann vorzugsweise eine druckfähige Paste eingesetzt werden, die als elektrochemische Aktivkomponente Braunstein ($MnO_2$) sowie weiterhin Ruß und/oder Graphit als Leitmaterial sowie einen geeigneten Binder und ein geeignetes Lösungsmittel aufweist.

Zur Herstellung der negativen Elektrode einer Zink-Luft-Zelle kann prinzipiell die gleiche Zinkpaste Verwendung finden wie bei der Herstellung der negativen Elektrode der erwähnten Zink-Metalloxid-Zelle. Die positive Elektrode einer Zink-Luft-Zelle bzw. die Sauerstoffkathode kann grundsätzlich gleichfalls aus einer druckfähigen Paste gebildet werden. Diese umfasst gegebenenfalls als elektrochemische Aktivkomponente ein elektrokatalytisch aktives Material wie beispielsweise Platin oder Palladium oder beispielsweise Manganoxid, sowie Ruß und/oder Graphit als Leitmaterial sowie, soweit erforderlich, einen geeigneten Binder, beispielsweise einen partikulären Kunststoffbinder. Geeignete Materialien zur Herstellung einer Sauerstoffkathode sind beispielsweise aus der DE 37 220 19 A1, auf die diesbezüglich vollumfänglich Bezug genommen wird, bekannt.

Bei dem erfindungsgemäßen Verfahren kann es vorgesehen sein, dass nur die elektrischen Leiter und die Elektroden und gegebenenfalls der Elektrolyt, aber nicht der gegebenenfalls verwendete schichtförmige Separator mittels eines Druckverfahrens gebildet werden. In dieser Ausgestaltung kann der Separator, der insbesondere als poröses Flächengebilde realisiert ist, als separate Komponente bereitgestellt werden.

Auch die Aufbringung bzw. das Tränken der negativen Elektrode und der positiven Elektrode mit einem flüssigen Elektrolyten kann mittels eines Druckverfahrens erfolgen.

Zweckmäßigerweise wird das gedruckte Energiespeicherelement mit einem Gehäuse abgeschlossen.

Das Gehäuse des Energiespeicherelements kann insbesondere aus einem ersten Gehäuseteil und einem zweiten Gehäuseteil gefertigt sein. Es ist aber beispielsweise auch möglich, die beiden Gehäuseteile beispielsweise durch Knicken oder Falten aus einem geeigneten Gehäusematerial zu bilden, so dass die Gehäuseteile über eine Knick- oder Faltlinie miteinander verbunden sind.

Das erste Gehäuseteil und das zweite Gehäuseteil können beispielsweise bevorzugt an ihren Rändern über einen umlaufenden Siegelrahmen miteinander verbunden sein. Bezüglich weiterer geeigneter Möglichkeiten für die Herstellung des diagnostischen Pflasters und insbesondere für das Drucken des Energiespeicherelements für das diagnostische Pflaster wird darüber hinaus auf die EP 3 477 727 A1 verwiesen, auf die diesbezüglich vollumfänglich Bezug genommen wird.

Bezüglich der weitergehenden Ausgestaltung der einzelnen Elemente des diagnostischen Pflasters bei dessen Herstellung und den Möglichkeiten zur Herstellung insbesondere des gedruckten Energiespeicherelements wird auch auf die obige Beschreibung des diagnostischen Pflasters selbst verwiesen.

Die Erfindung umfasst ferner ein Verfahren zur Diagnose unter Verwendung des beschriebenen diagnostischen Pflasters. Mit diesem Verfahren können verschiedene diagnostisch relevante Körperparameter erfasst werden, insbesondere die Sauerstoffsättigung im Blut eines Probanden und/oder die Pulsfrequenz und/oder die Herzfrequenz und/oder verschiedene Blutgaswerte, insbesondere die Kohlenstoffmonoxid-Beladung im Blut und/oder der $CO_2$-Gehalt im Blut und/oder der $HCO_3$-Gehalt im Blut und/oder verschiedene Elektrolyten in Körperflüssigkeiten und insbesondere im Blut, beispielsweise Natrium, Kalium oder Magnesium in Blut oder in Gewebsflüssigkeiten oder in Flüssigkeiten auf der Körperoberfläche. Weiterhin kann dieses Verfahren für eine Analyse einer Laktatkonzentration in Körperflüssigkeiten eingesetzt werden. Weiterhin und besonders bevorzugt kann dieses Verfahren für eine Analyse des Gewebezuckergehalts verwendet werden, woraus unmittelbar Rückschlüsse auf den Blutzuckergehalt, insbesondere im Zusammenhang mit einer Überwachung eines Diabetespatienten, gezogen werden können.

Schließlich umfasst die Erfindung ein Verfahren zur Erstellung eines Elektrokardiogramms. Dieses Verfahren umfasst die Anbringung von mindestens zwei, vorzugsweise drei oder vier (bei einem 3er-RKG) oder zehn (bei einem 12er-EKG), diagnostischen Pflastern gemäß der obigen Beschreibung an definierten Positionen am Körper des Probanden. Hierbei sind die diagnostischen Pflaster mit einer Spannungsmess-Sensorik ausgestattet. Bei diesem Verfahren ist es weiterhin vorgesehen, dass die Position der einzelnen diagnostischen Pflaster definiert wird und eine Auswertung der erfassbaren Sensordaten in Bezug zu der Position der einzelnen diagnostischen Pflaster erfolgt. Im Übrigen erfolgt die Auswertung der Sensordaten in an sich bekannter Weise.

Weitere Merkmale und Aspekte der Erfindung ergeben sich aus der nachfolgenden Beschreibung von Ausführungsbeispielen im Zusammenhang mit der Zeichnung. Hierbei können die einzelnen Merkmale jeweils für sich oder in Kombination miteinander verwirklicht sein.

KURZBESCHREIBUNG DER ZEICHNUNG

Die Figur zeigt eine bevorzugte Ausführungsform eines erfindungsgemäßen diagnostischen Pflasters im Querschnitt (Teilfigur 1A) und in Aufsicht (Teilfigur 1B).

DETAILLIERTE BESCHREIBUNG DER AUSFÜHRUNGSBEISPIELE

Die **Figur** illustriert in schematischer Weise ein erfindungsgemäßes diagnostisches Pflaster 10, wobei in der Teilfigur 1A ein Querschnitt und in der Teilfigur 1B eine Aufsicht gezeigt ist. Das diagnostische Pflaster 10 umfasst ein flächiges Substrat 11, das zur Aufbringung auf die Körperoberfläche eines Probanden vorgesehen ist. Bei dem flächigen Substrat 11 handelt es sich insbesondere um einen Schaumstoffträger, der auf der Seite, die für die Aufbringung auf der Körperoberfläche des Probanden vorgesehen ist, mit einer Kleberschicht 12 versehen ist. Zum Schutz der Kleberschicht 12 ist vor der Aufbringung auf die Körperoberfläche des Probanden auf der Unterseite des Pflasters eine Kleberschutzschicht 13 vorgesehen, die in an sich bekannter Weise abgezogen werden kann, bevor das Pflaster 10 auf der Körperoberfläche platziert wird. Auf der Oberfläche des flächigen Substrats 11 befindet sich in einem zentralen Bereich 17 mindestens eine Sensorik-Einrichtung 14. Die Sensorik-Einrichtung 14 kann insbesondere mit optischen Sensormitteln ausgestattet sein, die beispielsweise eine Analyse der Sauerstoffsättigung im Blut und/oder eine Pulsmessung und/oder eine Erfassung der Herzfrequenz und/oder eine Analyse der Kohlenstoffmonoxid-Beladung im Blut oder die Erfassung anderer diagnostisch interessanter Sensordaten vornehmen kann.

Weiterhin kann es sich bei der Sensorik-Einrichtung 14 auch um eine Spannungsmess-Sensorik handeln, mit der Daten erfasst werden können, die als Elektrokardiogramm ausgewertet werden können. Insbesondere für solche Anwendungen werden mehrere derartiger diagnostischer Pflaster 10 eingesetzt, beispielsweise drei oder vier oder zehn Pflaster, die an definierten Positionen am Körper platziert werden.

Weiterhin ist auf der Oberfläche des Substrats 11 eine Einrichtung zur drahtlosen Datenübertragung 15 angeordnet. Bei dieser Elektronik-Einheit kann es sich zum Beispiel um einen Mikrochip handeln, wie es sich dem Fachmann erschließt.

Weiterhin umfasst das diagnostische Pflaster 10 im zentralen Bereich 17 ein gedrucktes Energiespeicherelement 16, das für die Energieversorgung der Sensorik-Einrichtung 14 und der Einrichtung 15 zur drahtlosen Datenübertragung sorgt. Bei dem gedruckten Energiespeicherelement handelt es sich insbesondere um ein primäres Energiespeicherelement, also um ein nicht wiederaufladbares Energiespeicherelement. In besonders bevorzugten Ausgestaltungen wird hierfür eine Zink-Metalloxid-Zelle oder eine Zink-Luft-Zelle verwendet.

In Bezug auf die Kleberschicht 12 kann es vorgesehen sein, dass die Unterseite des zentralen Bereichs 17 von der Kleberschicht ausgespart ist, um insbesondere eine Interaktion der Sensorik mit dem Kleber, beispielsweise eine Absorption der verwendeten elektromagnetischen Strahlung in der Kleberschicht, zu vermeiden.

## Patentansprüche

1. Diagnostisches Pflaster mit den folgenden Merkmalen:

   a. das Pflaster umfasst ein flächiges Substrat zur Aufbringung auf die Körperoberfläche eines Probanden,
   b. das Pflaster umfasst mindestens eine Sensorik-Einrichtung,
   c. das Pflaster umfasst mindestens eine Einrichtung zur drahtlosen Datenübertragung,
   d. das Pflaster umfasst mindestens ein gedrucktes Energiespeicherelement.

2. Diagnostisches Pflaster nach Anspruch 1 mit den folgenden zusätzlichen Merkmalen:

   a. das gedruckte Energiespeicherelement umfasst eine negative Elektrode, die als Elektrodenaktivmaterial metallisches Zink oder eine metallische Zinklegierung aufweist,
   b. das gedruckte Energiespeicherelement umfasst eine positive Elektrode, die als Elektrodenaktivmaterial mindestens ein Metalloxid und/oder einen Katalysator, der eine Reduktion von Luftsauerstoff ermöglicht, aufweist,
   c. das gedruckte Energiespeicherelement umfasst einen wässrigen Elektrolyten.

3. Diagnostisches Pflaster nach Anspruch 2 mit den folgenden zusätzlichen Merkmalen:

   a. die negative Elektrode umfasst ein erstes elastisches Bindemittel oder Bindemittelgemisch,
   b. die positive Elektrode umfasst ein zweites elastisches Bindemittel oder Bindemittelgemisch,
   c. das Metalloxid der positiven Elektrode ist Manganoxid und/oder Silberoxid,
   d. die positive Elektrode umfasst mindestens ein Leitfähigkeitsadditiv, insbesondere ein Kohlenstoff-basiertes Leitfähigkeitsadditiv,
   e. der wässrige Elektrolyt ist ein nicht-alkalischer Elektrolyt,
   f. der wässrige Elektrolyt umfasst mindestens ein chloridbasiertes Leitsalz, insbesondere Zinkchlorid und/oder Ammoniumchlorid.

4. Diagnostisches Pflaster nach Anspruch 2 mit den folgenden zusätzlichen Merkmalen:

   a. die negative Elektrode umfasst ein erstes elastisches Bindemittel oder Bindemittelgemisch,
   b. die positive Elektrode umfasst ein zweites elastisches Bindemittel oder Bindemittelgemisch,
   c. der Katalysator, der eine Reduktion von Luftsauerstoff ermöglicht, ist Manganoxid und/oder ein Edelmetall,
   d. die positive Elektrode umfasst mindestens ein Leitfähigkeitsadditiv, insbesondere ein Kohlenstoff-basiertes Leitfähigkeitsadditiv,
   e. der wässrige Elektrolyt ist ein alkalischer Elektrolyt.

5. Diagnostisches Pflaster nach einem der Ansprüche 2 bis 4 mit einem der folgenden zusätzlichen Merkmale:

   a. das gedruckte Energiespeicherelement ist ein koplanares Energiespeicherelement mit der negativen Elektrode und der positiven Elektrode,
   b. das gedruckte Energiespeicherelement wird von einem Schichtstapel gebildet, wobei der Schichtstapel die negative Elektrode und die positive Elektrode sowie mindestens einen Separator umfasst.

6. Diagnostisches Pflaster nach einem der vorhergehenden Ansprüche mit mindestens einem der folgenden zusätzlichen Merkmale:

   a. die Einrichtung zur drahtlosen Datenübertragung basiert auf einem NFC-Übertragungsstandard,
   b. die Einrichtung zur drahtlosen Datenübertragung basiert auf einem Mobilfunkstandard, insbesondere auf einem LTE-Standard,
   c. die Einrichtung zur drahtlosen Datenübertragung basiert auf einem Bluetooth-Standard,
   d. die Einrichtung ist zur drahtlosen Übertragung von Daten über eine Reichweite von 5 cm bis 20 m, insbesondere von 10 cm bis 10 m, eingerichtet.

7. Diagnostisches Pflaster nach einem der vorhergehenden Ansprüche mit mindestens einem der folgenden zusätzlichen Merkmale:

   a. die Sensorik-Einrichtung umfasst eine optische Sensorik,
   b. die Sensorik-Einrichtung umfasst eine Spannungsmess-Sensorik,
   c. die Sensorik-Einrichtung umfasst mindestens eine Temperatursensorik.

**8.** Diagnostisches Pflaster nach Anspruch 7 mit mindestens einem der folgenden zusätzlichen Merkmale:

> a. die optische Sensorik ist zur Analyse von Blutgaswerten eingerichtet,
> b. die optische Sensorik ist zur Analyse der peripheren kapillaren Sauerstoffsättigung eingerichtet,
> c. die optische Sensorik ist zur Pulsmessung eingerichtet,
> d. die optische Sensorik ist zur Pulsoxymetrie eingerichtet,
> e. die optische Sensorik ist zur Erfassung der Herzfrequenz eingerichtet,
> f. die optische Sensorik ist zur Analyse der Kohlenstoffmonoxid-Beladung im Blut eingerichtet,
> g. die optische Sensorik ist zur Analyse des $CO_2$-Gehalts und/oder des $HCO_3$-Gehatts im Blut eingerichtet,
> h. die optische Sensorik ist zur Analyse des Gewebezuckergehaltes eingerichtet,
> i. die optische Sensorik ist zur Analyse einer Metallionenkonzentration in Körperflüssigkeiten eingerichtet,
> j. die optische Sensorik ist zur Analyse einer Laktatkonzentration in Körperflüssigkeiten eingerichtet,
> k. die optische Sensorik ist zur Temperaturmessung eingerichtet.

**9.** Diagnostisches Pflaster nach Anspruch 7 oder Anspruch 8 mit den folgenden zusätzlichen Merkmalen:

> a. die optische Sensorik weist mindestens eine Lichtwellen-emittierende Einheit auf,
> b. die optische Sensorik weist mindestens eine Einheit zum Empfangen von Lichtwellen und zur Umwandlung der empfangenen Lichtwellen in ein elektrisches Signal auf.

**10.** Diagnostisches Pflaster nach Anspruch 9 mit einem der folgenden zusätzlichen Merkmale:

> a. die optische Sensorik weist zwei Lichtwellen-emittierende Einheiten auf, wobei die einzelnen Lichtwellen-emittierenden Einheiten Lichtwellen mit unterschiedlicher Wellenlänge emittieren,
> b. die optische Sensorik weist drei Lichtwellen-emittierende Einheiten auf, wobei die einzelnen Lichtwellen-emittierenden Einheiten Lichtwellen mit unterschiedlicher Wellenlänge emittieren,
> c. die optische Sensorik weist vier bis sieben Lichtwellen-emittierende Einheiten auf, wobei die einzelnen Lichtwellen-emittierenden Einheiten Lichtwellen mit unterschiedlicher Wellenlänge emittieren,
> d. die Lichtwellen-emittierende Einheit ist zur Emission von Laserstrahlen eingerichtet,
> e. die Lichtwellen-emittierende Einheit ist zur Emission von Fluoreszenzstrahlen eingerichtet.

**11.** Diagnostisches Pflaster nach einem der Ansprüche 7 bis 10 mit dem folgenden zusätzlichen Merkmal:

> a. die Spannungsmess-Sensorik ist zur Erfassung eines Elektrokardiogramms eingerichtet.

**12.** Diagnostisches Pflaster nach einem der vorhergehenden Ansprüche mit dem folgenden zusätzlichen Merkmal:

> a. das diagnostische Pflaster weist mindestens eine Abschirmschicht zur Abschirmung von elektromagnetischer Strahlung auf.

**13.** Diagnostisches Pflaster nach einem der vorhergehenden Ansprüche mit dem folgenden zusätzlichen Merkmal:

> a. das diagnostische Pflaster weist mindestens eine Elektronik-Einheit zur Datenverarbeitung auf.

**14.** Verfahren zur Herstellung eines diagnostischen Pflaster gemäß einem der Ansprüche 1 bis 13 mit den folgenden Schritten:

> a. es wird ein flächiges Substrat bereitgestellt, das zur Aufbringung auf die Körperoberfläche eines Probanden geeignet ist,
> b. auf das flächige Substrat wird mindestens eine Sensorik-Einrichtung aufgebracht,
> c. auf das flächige Substrat wird mindestens eine Einrichtung zur drahtlosen Datenübertragung aufgebracht,
> d. auf das flächige Substrat wird mindestens ein Energiespeicherelement aufgedruckt oder auf dem flächigen Substrat wird ein gedrucktes Energiespeicherelement angeordnet.

**15.** Verfahren nach Anspruch 14, wobei das Aufdrucken des mindestens einen Energiespeicherelements mindestens einen der folgenden zusätzlichen Schritte umfasst:

> a. es wird ein erster elektrischer Leiter und ein zweiter elektrischer Leiter gedruckt,
> b. es wird eine negative Elektrode als Schicht auf den ersten elektrischen Leiter unter Verwendung einer Druckpaste, die partikuläres metallisches Zink oder eine partikuläre metallische Zinklegierung enthält, und die ein erstes elastisches Bindemittel oder Bindemittelgemisch enthält, gedruckt,

c. es wird eine positive Elektrode als Schicht auf den zweiten elektrischen Leiter unter Verwendung einer Druckpaste, die ein partikuläres Metalloxid und/oder einen Katalysator, der eine Reduktion von Luftsauerstoff ermöglicht, und ein zweites elastisches Bindemittel oder Bindemittelgemisch enthält, gedruckt,

d. es wird die negative Elektrode und die positive Elektrode mit einem flüssigen Elektrolyten getränkt.

10

17

11 12 16 14 15 13

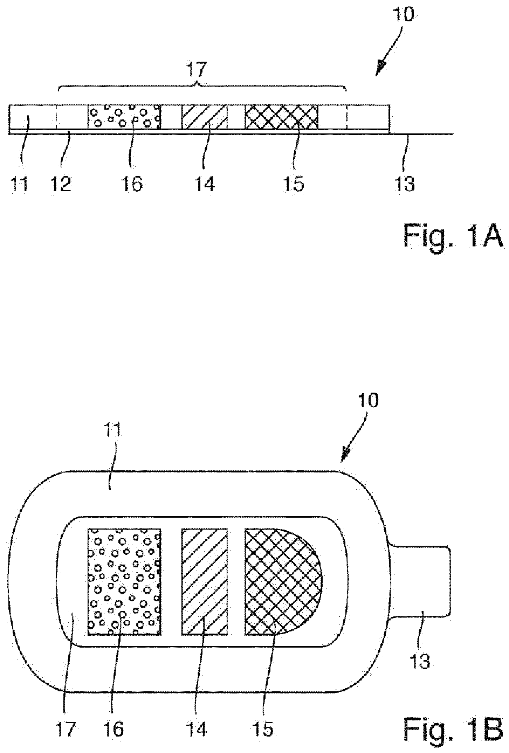

# Fig. 1A

10

11

17 16 14 15

13

# Fig. 1B

Europäisches
Patentamt

European
Patent Office

Office européen
des brevets

**EUROPÄISCHER RECHERCHENBERICHT**

Nummer der Anmeldung

EP 20 17 1431

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2019/032245 A1 (BLUE SPARK TECH INC [US]) 14. Februar 2019 (2019-02-14) <br> * Abbildungen 1, 3, 5-8, 10, 13 * <br> * Absatz [0052] - Absatz [0053] * <br> * Absatz [0088] * <br> * Absatz [0093] - Absatz [0114] * <br> * Absatz [0131] - Absatz [0143] * <br> ----- | 1-15 | INV. <br> A61B5/00 <br> A61B5/0205 <br> G16H10/00 <br> A61F13/00 <br> H01M4/00 |
| X | US 2014/121557 A1 (GANNON JOHN [US] ET AL) 1. Mai 2014 (2014-05-01) <br> * Abbildungen 1-2 * <br> * Absatz [0060] * <br> ----- | 1-15 | |
| X | US 2016/183794 A1 (GANNON JOHN [US] ET AL) 30. Juni 2016 (2016-06-30) <br> * Abbildung 1 * <br> * Absatz [0075] * <br> ----- | 1-15 | |
| X | US 2017/156594 A1 (STIVORIC JOHN M [US] ET AL) 8. Juni 2017 (2017-06-08) <br> * Absatz [0109] * <br> * Absatz [0140] - Absatz [0141] * <br> * Abbildung 8 * <br> ----- | 1-15 | RECHERCHIERTE SACHGEBIETE (IPC) |
| A | EP 3 276 706 A1 (VARTA MICROBATTERY GMBH [DE]) 31. Januar 2018 (2018-01-31) <br> * Anspruch 3; Abbildung 3 * <br> ----- | 5 | A61B <br> G16H <br> A61F <br> H01M |
| A | US 2015/024247 A1 (LOCKETT VERA [US] ET AL) 22. Januar 2015 (2015-01-22) <br> * Absatz [0101] - Absatz [0153] * <br> ----- | 1-15 | |

Der vorliegende Recherchenbericht wurde für alle Patentansprüche erstellt

| Recherchenort | Abschlußdatum der Recherche | Prüfer |
|---|---|---|
| Den Haag | 31. Juli 2020 | Knoop, Jan |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 20 17 1431

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-07-2020

| Im Recherchenbericht angeführtes Patentdokument | Datum der Veröffentlichung | Mitglied(er) der Patentfamilie | Datum der Veröffentlichung |
|---|---|---|---|
| WO 2019032245 A1 | 14-02-2019 | AU 2018314163 A1<br>EP 3664701 A1<br>US 2019046033 A1<br>WO 2019032245 A1 | 27-02-2020<br>17-06-2020<br>14-02-2019<br>14-02-2019 |
| US 2014121557 A1 | 01-05-2014 | CN 104936513 A<br>DE 13852079 T1<br>EP 2914167 A1<br>HK 1210402 A1<br>JP 6316305 B2<br>JP 2016505808 A<br>KR 20150066560 A<br>US 2014121557 A1<br>US 2018028073 A1<br>WO 2014070254 A1 | 23-09-2015<br>19-11-2015<br>09-09-2015<br>22-04-2016<br>25-04-2018<br>25-02-2016<br>16-06-2015<br>01-05-2014<br>01-02-2018<br>08-05-2014 |
| US 2016183794 A1 | 30-06-2016 | US 2016183794 A1<br>US 2017332904 A1 | 30-06-2016<br>23-11-2017 |
| US 2017156594 A1 | 08-06-2017 | KEINE | |
| EP 3276706 A1 | 31-01-2018 | KEINE | |
| US 2015024247 A1 | 22-01-2015 | CN 105379000 A<br>EP 3022794 A1<br>KR 20160032228 A<br>TW 201511398 A<br>US 2015024247 A1<br>US 2018034067 A1<br>WO 2015009867 A1 | 02-03-2016<br>25-05-2016<br>23-03-2016<br>16-03-2015<br>22-01-2015<br>01-02-2018<br>22-01-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202016003095 U1
- EP 1062906 A1
- EP 2561564 B1
- EP 3477727 A1
- EP 2960967 B1
- DE 3722019 A1